# EUROPÄISCHE PATENTSCHRIFT

(11) **EP 1 341 925 B1**
(45) Veröffentlichungstag und Bekanntmachung des Hinweises auf die Patenterteilung: **08.08.2007**
(21) Anmeldenummer: 01991674.1
(22) Anmeldetag: 20.12.2001
(51) Int. Cl.: C12P 17/16

(54) **MIKROBIOLOGISCHES VERFAHREN ZUR BIOSYNTHESE DER NATÜRLICHEN BLAU-VIOLETTEN FARBSTOFFE VIOLACEIN UND DESOXYVIOLACEIN**
MICROBIOLOGICAL METHOD FOR THE BIOSYNTHESIS OF NATURAL BLUE-VIOLET COLORANTS VIOLACEIN AND DESOXYVIOLACEIN
PROCEDE MICROBIOLOGIQUE DE BIOSYNTHESE DES COLORANTS NATURELS BLEU-VIOLETS VIOLACEINE ET DESOXYVIOLACEINE

(30) Priorität: 20.12.2000 DE 10063712
(43) Veröffentlichungstag der Anmeldung: 10.09.2003
(73) Patentinhaber: Stiftung Alfred-Wegener-Institut für Polar- und Meeresforschung, 27568 Bremerhaven (DE)
(72) Erfinder: TAN, Tjhing-Lok, 27607 Langen (DE); MONTFORTS, Franz-Peter, 28665 Lilienthal (DE); MEYER, Daniela, 28219 Bremen (DE)
(86) Internationale Anmeldenummer: PCT/DE2001/004900
(87) Internationale Veröffentlichungsnummer: WO 2002/050299

(56) Entgegenhaltungen:
- DE-A- 3 935 066
- DATABASE CA [Online] CHEMICAL ABSTRACTS SERVICE, COLUMBUS, OHIO, US; MCCARTHY, SUSAN A. ET AL: "Studies on marine purple bacteria. I. Production and isolation of purple pigment by Alteromonas luteoviolacea" retrieved from STN Database accession no. 103:19496 XP002215962 & NIPPON SUISAN GAKKAISHI (1985), 51(3), 479-84 ,
- WILLE ET AL: "A short synthesis of the bacterial pigments violacein and deoxyviolacein" SYNTHESIS, GEORG THIEME VERLAG. STUTTGART, DE, Nr. 5, 12. April 2001 (2001-04-12), Seiten 759-762, XP002201463 ISSN: 0039-7881
- PATENT ABSTRACTS OF JAPAN vol. 1999, no. 11, 30. September 1999 (1999-09-30) & JP 11 152687 A (NATIONAL INSTITUTE OF SERICULTUAL AND ENTOMOLOGICAL SCIENCE), 8. Juni 1999 (1999-06-08)
- PATENT ABSTRACTS OF JAPAN vol. 1998, no. 10, 31. August 1998 (1998-08-31) & JP 10 139612 A (NAKANIHON SENI KOGYO KYODO KUMIAI), 26. Mai 1998 (1998-05-26)
- PATENT ABSTRACTS OF JAPAN vol. 1998, no. 10, 31. August 1998 (1998-08-31) & JP 10 113169 A (NORIN SUISANSYO SANSHI KONCHU NOGYO GIJUTSU KENKYUSHO;KOJIMA ATSUSHI), 6. Mai 1998 (1998-05-06)
- DATABASE WPI Section Ch, Week 199924 Derwent Publications Ltd., London, GB; Class B02, AN 1999-278022 XP002216054 & BR 9 702 918 A (UNICAMP UNIV ESTADUAL CAMPINAS), 4. Mai 1999 (1999-05-04)

## Beschreibung

Die Erfindung bezieht sich auf ein mikrobiologisches Verfahren zur Biosynthese der natürlichen blau-violetten Farbstoffe Violacein und Desoxyviolacein aus einem pigmentbildenden Bakterium durch Kultivieren des Bakteriums, Abzentrifugieren der Zellmasse, Extrahieren eines Farbstoffrohextraktes aus der Zellmasse und Isolieren der Farbstoffe aus dem Farbstoffrohextrakt.

Farbstoffe sind in mehr als einer Hinsicht interessant für die Mikrobiologie und Chemie. Da die Fähigkeit, Pigmente zu produzieren, genetisch fixiert ist, ist die Bildung von Farbstoffen auch ein erster Hinweis auf die Mitbildung antibiotischer Wirkstoffe. Mikrobielle Pigmente weisen eine große strukturelle Vielfalt auf. So kann es sich um Derivate aus den Stoffklassen der Carotinoide, der Phenazinfarbstoffe, der Pyrrolfarbstoffe, der Azachinone usw. handeln.

In den Vereinigten Staaten von Amerika und in Japan wird mit thermophilen Bakterien aus Tiefseeschloten und mit symbiontischen Bakterien aus Invertebraten und Vertebraten bereits seit etwa 20 Jahren nach bioaktiven Naturstoffen gesucht, die biotechnologisch genutzt werden können. In Ländern der Europäischen Gemeinschaft wurde die Nutzbarkeit mariner Organismen für die pharmazeutische Industrie, für Aquakultur, für mikrobielle Reinigungen von ölkontaminierten Meeresregionen und für Vorgänge bei der Entstehung von Biofilmen und Bioadhäsionen erkannt. Erst seit 3 Jahren jedoch werden vermehrt auch Projekte mit marinen Mikroorganismen in einer mehr anwendungsorientierten Forschungsrichtung in Deutschland konzipiert. In der Textil-, Lebensmittel-, Kosmetik- und Pharmaindustrie wird eine Vielzahl von Farbstoffen eingesetzt. Hier ist in den letzten Jahren seitens der Verbraucher ein deutlicher Trend zur Bevorzugung von Produkten zu beobachten, welche natürliche Farbstoffe enthalten. Gelbe und rote Farbstoffe werden bereits heute im industriellen Maßstab aus pflanzlichen Rohstoffen gewonnen. Dagegen besteht in den genannten Industriezweigen immer noch ein hoher Bedarf an natürlichen blauen und violetten Farbstoffen. Die Suche nach einem Farbstoff aus diesem Spektrum gewinnt durch das lebensmittelrechtliche Verbot des rot-blauen Farbstoffs Monascin, isoliert aus *Monascus purpureus,* besondere Bedeutung. Eine Einordnung von Monascin als färbendes Lebensmittel ist wegen auftretender allergologischer Probleme nicht möglich. Ein mikrobiologisch gewonnener blau-violetter Farbstoff kann somit helfen, die entstandene Lücke zu schließen und ökonomisch hochinteressante Wege zu beschreiten.

Pigmente aus dem blau-violetten Spektrum werden unter anderem durch Mikroorganismen gebildet. Das Azachinon Indigoidin wird von mehreren Organismen *(Pseudomonas indigofera, Corynebacterium insidiosum, Arthrobacter arthrocyaneus* und *Arthrobacter polychromogenes)* gebildet und in das umgebende Medium ausgeschieden. Dieses ist für eine kontinuierliche Prozessführung und für eine vereinfachte Produktaufarbeitung von Vorteil. Hierdurch kann auf den Schritt des Zellaufschlusses verzichtet werden. Indigoidin ist bereits lebensmittelrechtlich zugelassen (E 132). Weitere zugelassene Lebensmittelfarbstoffe aus dem blauen Farbspektrum sind Patentblau V (E 131), Brillantblau FCF und Brillantschwarz (E 151). Diese Verbindungen weisen Absorptionsspektren im Bereich 570 nm bis 638 nm auf und eignen sich daher sehr gut für einen Einsatz in Prozessen der Lebensmitteltechnik.

Das blau-schwarze Pigment Violacein, ein Indolderivat, wird in der Literatur erstmals 1882 beschrieben. Damals wurde ein violetter Farbstoff aus einem Bakterium isoliert, das heute als *Chromobacterium violaceum* bekannt ist. Violacein kann bislang aus den Bakterienstämmen *Janthinobacterium lividum, Chromobacterium lividum* und *Alteromonas luteoviolacea* (vergleiche H. Laatsch et al., "Spectroscopic Properties of Violacein und Related Compounds : Crystal Structure of Tetramethylviolacein", J. Chem. Soc. Perkin Trans 2., 1984, pp. 13331 - 1339) isoliert werden. Die Struktur des Violaceins wurde 1960 durch Synthese bewiesen. Chemisch ist Violacein 3-[1,2-dihydro-5-(5-hydroxy-1H-indol-3-yl)-2-oxo-3-H-pyrrol-3-yliden]-1,3-dihydro-2H-indol-2-on mit der Summenformel C20-H13-N3-O3 (Molekulargewicht 343,33) charakterisiert. Die maximale Absorptionsbande liegt in methanolischer Lösung bei 570 nm. Violacein ist in Wasser nahezu unlöslich, jedoch löslich in Aceton, Ethanol und Dioxan. Violacein ist der Hauptbestandteil eines blauvioletten Pigmentes in *Chromobacterium violaceum,* ein aus Erde und Gewässer tropischer Gebiete erhältlicher Mikroorganismus, wobei als Nebenbestandteil Desoxyviolacein mit identischem Strukturaufbau, aber einem Sauerstoffatom weniger als Violacein auftritt (Summenformel C20-H13-N3-02). Sogenanntes "natives Violacein" besteht aus einer Mischung von Violacein mit bis zu 10 % Desoxyviolacein. Das Violacein dient der Zelle zum Schutz vor Strahlung und zur Regelung der Tryptophankonzentration unter das toxische Niveau. Violacein besitzt antibiotische, antivirale und antitumorale Eigenschaften, zeigt aber keine zytotoxische oder pathogene Wirkung. In neueren Untersuchungen wurde Violacein außerdem zur Färbung von Textilien eingesetzt, wobei nicht nur auf natürlichen Stoffen, wie Seide, Wolle und Baumwolle, sondern auch auf synthetischen Fasern, wie Nylon, gute Färberesultate erzielt wurden (vgl. Shirata et al. "Isolation of Bacteria Producing Bluish-Purple Pigment and Use for Dyeing", Jpn. Agr. Res. Q 2000, 34(2), pp. 131-140, mit einer Pigmentgewinnung aus *Janthinobacterium lividum).*

Die Biosynthese aus bekannten Mikroorganismen wird in verschiedenen Druckschriften beschrieben. Ein Verfahren zur Herstellung nativen Violaceins, ausgehend von dem pigmentbildenden Bakterium *Chromobacterium violaceum,* für eine Verwendung zur Behandlung von Viruserkrankungen wird in der DE3935066 beschrieben. Die Biosynthese basiert auf den Verfahrensschritten "Kultivieren des Bakteriums", "Abzentrifugieren der Zellmasse", "Extrahieren eines Farbstoffrohextraktes aus der Zellmasse" und "Isolieren des Farbstoffs aus dem Farbstoffrohextrakt". Die als Ausgangsmaterial verwendeten Bakterien werden auf festen oder flüssigen Nährmedien kultiviert. Vorzugsweise werden die Bakterien in flüssigen Nährmedien gezüchtet, da sie aus dem Nährmedium dann durch Zentrifugieren leicht abgetrennt werden können. Die Bakterienkultivierung kann in Fermentern mit unterschiedlichen Verfahrensparametern erfolgen. Bei dem bekannten Verfahren wird der nach Bebrütung gewachsene Bakterienrasen abgelöst und gefriergetrocknet. Die Extraktion des Roh-Violaceins erfolgt mit Ethanol im Soxhlet. Das Ethanol wird im Vakuum abdestilliert. Zur Isolierung des Violaceins wird das Roh-Violacein zweimal mit n-Heptan extrahiert und dann abgefiltert. Der Rückstand wird in einem Gemisch aus Chloroform / Aceton / Pyridin 50:40:10 aufgelöst. Das Farbstoffgemisch wird dann mittels Kieselgel-Dünnschichtchromatographie aufgetrennt und gereinigt.

Aus dem **Aufsatz** "Production, extraction and purification of violacein : an antibiotic pigment produced bei *Chromobacterium violaceum* ( Rettori, Duran, World Journal of Microbiology & Biotechnology 14 (1998) pp. 685-688) ist eine einfache Methode zur Erzielung hochreinen Violaceins bekannt. Zur Kultivierung des Bakteriums werden unsterile Baumwollläppchen mit einer Suspensionskultur des pigmentbildenden *Chromobacterium violaceum* CCT 3496 geimpft und in einem stark durchlüfteten Brutschrank gelagert, wo ein starkes Bakterienwachstum eintritt. Die Zellmasse wird anschließend aus den Baumwollläppchen mit Lösungsmittel ausgewaschen und gefiltert, woraus ein Farbstoffrohextrakt gewonnen wird. Daraus wird durch mehrfache Reinigung mit unterschiedlichen Verfahren (Soxhlet, HPLC) hochreines Violacein als Farbstoff isoliert. Die Ausbeute ist jedoch relativ gering und bewegt sich im mg-Bereich pro Liter Nährmedium.

Aus dem **Aufsatz** "Biosynthesis of Violacein : a Novel Rearrangement in Tryptophan Metabolism with a 1,2-Shift of the Indole Ring" (T. Hoshino et al., Agric. Biol. Chem. 51 (3), 1987, pp. 965-968) ist es bekannt, Violacein aus L-Tryptophan als biosynthetischem Ausgangsmaterial herzustellen, wobei das Kohlenstoffgerüst des Pyrrolinon-Rings durch die Kondensation der Seitengruppen zweier Tryptophanmoleküle unter 1,2-shift des Indol-Rings gebildet wird. Gegenüber der Herstellung von Violacein aus einer reinen Suspensionskultur von *Chromobacterium violaceum* JCM 1249 kann zwar durch eine Zugabe von L-Tryptophan in die Suspensionskultur eine Ausbeutesteigerung um ca. das 1,5fache erreicht werden. Dies entspricht aber bei einer üblichen Ausbeute im mg-Bereich pro Liter Nährlösung immer noch einer sehr geringen Ausbeute.

Zusammenfassend gesehen sind die bekannten Verfahren zur Biosynthese von Violacein und Desoxyviolacein nicht dazu geeignet, diese Farbstoffe in größeren Mengen zur Verfügung zu stellen. Für Anwendungen im Pharmabereich ist dies auch nicht unbedingt erforderlich, da hier bereits kleinste Mengen wirkungsvoll eingesetzt werden können. Bei anderen Anwendungen kann der industrielle Bedarf an den blau-violetten Farbstoffen jedoch sehr groß sein. Werden diese nach den bekannten Verfahren synthetisiert, sind große Mengen an eingesetzter Suspensionskultur erforderlich, deren Bearbeitung dann entsprechend geräte- und zeitaufwändig ist. **Aufgabe** für die vorliegende Erfindung ist es daher, ein Verfahren der eingangs beschriebenen Art anzugeben, mit dem erheblich größere Ausbeuten an Violacein und Desoxyviolacein als mit den bekannten Verfahren auf ökonomischem Wege und mit einfachen prozesstechnischen Mitteln erzielbar sind.

Als Lösung dafür ist bei dem erfindungsgemäßen mikrobiologischen Verfahren zur Biosynthese der natürlichen blau-violetten Farbstoffe Violacein und Desoxyviolacein aus einem pigmentbildenden Bakterium deshalb vorgesehen, dass als pigmentbildendes Bakterium das neu entdeckte marine Sedimentbakterium *Pseudoalteromonas spezies* Stamm "Black Beauty" (ersthinterlegt nach dem Budapester Vertrag für die Zwecke von Patentverfahren unter der Eingangsnummer DSM 13623 bei der Deutschen Sammlung von Mikroorganismen und Zellkulturen GmbH, Braunschweig, Deutschland) eingesetzt wird, wobei als geringfügiges Nebenprodukt ein gelber Farbstoff auftritt, der durch eine weitere Extraktion des Farbstoffrohextraktes mit Dichlormethan separiert wird.

Vorteilhafte Weiterbildungen des erfindungsgemäßen Verfahrens bezüglich der einzelnen Verfahrensschritte "Kultivieren des Bakteriums", "Abzentrifugieren der Zellmasse", "Extrahieren eines Farbstoffrohextraktes aus der Zellmasse" und "Isolieren der Farbstoffe aus dem Farbstoffrohextrakt" sind den Unteransprüchen und der nachfolgenden Beschreibung zu entnehmen. Die mit dem erfindungsgemäßen Verfahren prozesstechnisch einfach und ökonomisch biosynthetisierten blau-violetten Farbstoffe Violacein und Desoxyviolacein können bei verbraucher- und umweltfreundlichen Produkten aus dem Bereich der Lebensmittel-, Textil-, Pharma- oder Spielzeugindustrie eingesetzt werden.

Eine lebende Bakterienkultur wurde bei der Internationalen Hinterlegungsstelle DSMZ - Deutsche Sammlung von Mikroorganismen und Zellkulturen GmbH, Braunschweig - nach dem Budapester Vertrag über die internationale Anerkennung der Hinterlegung von Mikroorganismen für die Zwecke von Patentverfahren hinterlegt. Mit dem Datum vom 28.07.2000 gingen der Anmelderin von der DSMZ eine Empfangsbestätigung bei Ersthinterlegung, ausgestellt gemäß Regel 7.1, und eine Lebensfähigkeitsbescheinigung, ausgestellt gemäß Regel 10.2, zu. Damit ist das neu entdeckte marine Sedimentbakterium *Pseudoalteromonas spezies* Stamm "Black Beauty" unter der Eingangsnummer DSM 13623 in seinen Anwendungen für die Anmelderin geschützt. Mit vorliegendem erfindungsgemäßem Verfahren wird die Erzeugung von zwei blau-violetten Farbstoffen unter Einsatz dieses Sedimentbakteriums beansprucht. Nach Kenntnis der Anmelderin ist es das erste Mal, dass für Anwendungen in Industrien (Pigment- und Lebensmittelindustrien) mit einem hohen Bedarf an natürlichen blauen Farbstoffen ein mariner Mikroorganismus zur Verfügung gestellt wird. Farbstoffe aus marinen Bakterien sind bislang kaum untersucht worden, sodass hier ein größeres Potenzial für die Naturstoffchemie zu sehen ist.

Die mit dem erfindungsgemäßen Verfahren erzielbare Ausbeute an Violacein mit dem marinen Sedimentbakterium *Pseudoalteromonas spezies* Stamm "Black Beauty" ist in einem Bereich des Dreizehnfachen höher als mit *Chromobacterium violaceum.* Beispielsweise erhält man aus 38,97 g "Black Beauty"-Bakterien-Feuchtmasse durch mehrmalige Extraktion mit heißem Methanol eine stark violett gefärbte Lösung, aus der sich nach Entfernung des Lösungsmittels dann 2,10 g eines tiefschwarzen Rohextraktes isolieren lassen. Gegenüber den bekannten Verfahren hat damit das erfindungsgemäße Verfahren, das auf der Biosynthese der blauen Farbstoffe aus "Black Beauty" basiert, einen sehr hohen Ausbeutefaktor. Das marine Sedimentbakterium "Black Beauty" weist also einen sehr hohen Pigmentanteil auf, der es zur Produktion der natürlichen blau-violetten Farbstoffe Violacein und Desoxyviolacein besonders geeignet macht. Die Pigmentausbeute ist darüber hinaus durch Parameteroptimierungen bei den verschiedenen Verfahrensschritten (Verweilzeiten, Lösungsmittelverhältnisse etc.), insbesondere bei der Kultivierung der Bakterien (Nährmedium, Schüttelfrequenz, Sauerstoffeintrag, pH-Wert, Salzgehalt, Temperatur etc.), noch steigerungsfähig. Insgesamt gesehen ergeben sich bei dem erfindungsgemäßen Verfahren bei einer moderaten Prozessführung und einem relativ geringen Zeitaufwand große ökonomische Vorteile, insbesondere in Hinblick auf großtechnische Nutzungen. Aufgrund der humanfreundlichen Eigenschaften des Violaceins ist es in diesem Zusammenhang besonders sinnvoll, wenn die biotechnologisch hergestellten Farbstoffe in Industriezweigen mit einem großen Bedarf an umweltfreundlichen blauen Farbstoffen eingesetzt werden, insbesondere Lebensmittel-, Spielzeug und Textilindustrie.

Dünnschichtchromatographische Untersuchungen zeigen, dass sich der aus dem marinen Sedimentbakterium "Black Beauty" gewonnene Farbstoffrohextrakt aus drei Pigmentfraktionen zusammensetzt: ein "schwacher", unpolarer, gelber Farbstoff, ein weiterer "schwacher", wenig polarer, violetter Farbstoff sowie ein "sehr starker", polarer, tief violett gefärbter Farbstoff. Der tief violett gefärbte Farbstoff macht dabei ca. 90% des Farbstoffrohextraktes aus. Strukturanalytische Untersuchungen mittels hochauflösender Massenspektrometrie und mehrdimensionaler NMR-Spektroskopie ergeben, dass es sich bei dem tief violett gefärbten Farbstoff um Violacein und bei dem violetten Farbstoff um Desoxyviolacein handelt. Der gelbe Farbstoff, der im Rohstoffextrakt als geringfügiges Nebenprodukt mit einem Anteil unter 2% auftritt, weist als Hauptkomponente Palmitoleinsäure auf. Hierbei handelt es sich um eine Fettsäure, die sowohl in pflanzlichen als auch in tierischen Fetten und Ölen als untergeordnete Nebenkomponente vorkommt. Der gelbe Farbstoff ist jedoch nicht Gegenstand der vorliegenden Erfindung und bedarf weiterer Strukturuntersuchungen. Für die Erfindung ist es wichtig, dass sein Anteil am Rohstoffextrakt nur sehr gering ist, sodass er die Ausbeute an den blau-violetten Farbstoffen kaum mindert, und dass er leicht aus dem Rohstoffextrakt zu entfernen ist, um daraus problemlos die beiden blauvioletten Farbstoffe gewinnen zu können.

Im Folgenden werden die einzelnen Schritte des erfindungsgemäßen Verfahrens in beispielhaften Ausführungsvarianten näher beschrieben. Grundsätzlich sind jedoch auch andere Parametervariationen möglich, die aus gattungsgemäßen Verfahren (vgl. zitierten Stand der Technik, beispielsweise DE3813465) bekannt sind, da es sich bei den einzelnen Verfahrensschritten letztendlich um solche von ganz allgemeiner Natur, dem Durchschnittsfachmann vertrauten Schritten handelt, wodurch sich auch die Verfahrenseinfachheit begründet.

### • Kultivierung der Bakterien

Als Ausgangskultur zur Synthese der Biomasse wurde eine seit 1985 bei -80°C im Alfred-Wegener-Institut für Polar- und Meeresforschung, Bremerhaven, Deutschland, aufbewahrte Bakteriendauerkultur (Fundstelle in diesem Falle aus dem Borkumer Watt, ersthinterlegt unter der Eingangsnummer DSM 13623 bei der Deutschen Sammlung von Mikroorganismen und Zellkulturen GmbH, Braunschweig, Deutschland)) aufgetaut und in mariner Kulturbrühe in vier 2 1-Erlenmeyerkolben mit je 1 I-Nährlösung bei 25°C auf einem Rundschüttler (100 bis 110 Upm) kultiviert. Nach vier Tagen wurden die Zellen durch Abzentrifugieren geerntet und die gewonnene Zellmasse für die Farbstoffextraktion verwendet.

### • Isolierung des Rohextraktes

38,97 g der Bakterienzellmasse (nass) werden in einen Erlenmeyerkolben überführt und mit ca. 250 ml heißem Methanol aufgeschlämmt. Dieses Gemisch lässt man etwa 2 Stunden stehen, bis sich die Zellen deutlich abgesetzt haben. Der Überstand wird abdekantiert und der Vorgang mehrmals wiederholt, bis die Zellen als farblose, graue Masse zurückbleiben. Diese Masse kann beispielsweise als Zusatz für Tierfuttermittel verwendet werden. Die vereinigten methanolischen Extrakte werden am Rotationsverdampfer vom Lösungsmittel befreit, und man erhält nach Trocknen im Ölpumpenvakuum 2,10 g eines tiefschwarzen Rohextraktes.

### • Isolierung des gelben Farbstoffes

514 mg des Rohstoffextraktes werden in 50 ml Dichlormethan suspendiert und unter Lichtausschluss und Argonatmosphäre ca. eine Stunde gerührt. Der verbleibende feste Rückstand (Farbstoffgemisch) wird abgetrennt, mit Dichlormethan gewaschen und im Ölpumpenvakuum getrocknet. Aus der Mutterlauge wird nach Entfernung des Lösungsmittels am Rotationsverdampfer 29,8 mg einer gelbbraunen, öligen Flüssigkeit isoliert, welche über Kieselgel mit Dichlormethan/ Essigester 1 +1 säulenchromatographiert wird. Man erhält 10,2 mg einer gelben Flüssigkeit, die in der Kälte kristallisiert.

### • Isolierung der beiden blau-violetten Farbstoffe

73,5 mg des von dem gelben Farbstoff abgetrennten Farbstoffgemisches werden in 15 ml Dichlormethan/Methanol (6+1) suspendiert und über Nacht stehen gelassen. Am nächsten Morgen wird der Überstand (13,5 mg) über 100 g Kieselgel mit Dichlormethan/Methanol (6+1) chromatographiert. Man erhält 0,4 mg Desoxyviolacein und 2,9 mg Violacein. Dieses kann für analytische Zwecke sowohl durch präparative HPLC als auch durch isotherme Umkristallisation aus Methanol/ n-Hexan zusätzlich gereinigt werden.

Die vorbeschriebenen Präparations- und Untersuchungsschritte sind Teil der Diplomarbeit "Isolierung und Strukturaufklärung von Pigmenten aus marinen Sedimentbakterien", die im Oktober 2000 der Institutsleitung für Organische Chemie, Fachbereich 2 der Universität Bremen, Deutschland, vorgelegt wurde. Der Diplomarbeit können im Bedarfsfall nach ihrer Veröffentlichung Anfang 2001 weitere Details bezüglich einzelner Prozess- und Untersuchungsparameter und -ergebnisse entnommen werden. Außerdem besteht ein Antrag auf Projektförderung im Zeitraum 01.04.2001 bis 31.03.2002 durch das BMBF, Biologie, Energie, Umwelt (BEO) beim Forschungszentrum Jülich, Bereich Meeres- und Polarforschung, Geowissenschaften, zum Thema "Marine Naturstoffforschung -Anwendung natürlicher Farbstoffe aus marinen Sedimentbakterien". In diesem Zusammenhang werden auch Industriepartner gesucht.

## Patentansprüche

1. Mikrobiologisches Verfahren zur Biosynthese der natürlichen blau-violetten Farbstoffe Violacein und Desoxyviolacein aus einem pigmentbildenden Bakterium durch Kultivieren des Bakteriums, Abzentrifugieren der Zellmasse, Extrahieren eines Farbstoffrohextraktes aus der Zellmasse und Isolieren der Farbstoffe aus dem Farbstoffrohextrakt,
**dadurch gekennzeichnet, dass**
als pigmentbildendes Bakterium das neu entdeckte marine Sedimentbakterium *Pseudoalteromonas spezies* Stamm "Black Beauty" (ersthinterlegt nach dem Budapester Vertrag für die Zwecke von Patentverfahren unter der Eingangsnummer DSM 13623 bei der Deutschen Sammlung von Mikroorganismen und Zellkulturen GmbH, Braunschweig, Deutschland) eingesetzt wird, wobei als geringfügiges Nebenprodukt ein gelber Farbstoff auftritt, der durch eine weitere Extraktion des Farbstoffrohextraktes mit Dichlormethan separiert wird.

2. Mikrobiologisches Verfahren nach Anspruch 1,
**dadurch gekennzeichnet, dass**
das Extrahieren des Farbstoffrohextraktes aus der Zellmasse durch Aufschlämmen mit heißem Methanol, Absetzen der Zellmasse in einem Zeitraum von ca. 2 Stunden und Abdekantieren des Überstandes mit den gebildeten methanolischen Extrakten in mehrmaliger Wiederholung, Entfernen des Lösungsmittels aus den vereinigten methanolischen Extrakten am Rotationsverdampfer und Trocknen im Ölpumpenvakuum erfolgt.

3. Mikrobiologisches Verfahren nach Anspruch 1 oder 2,
**dadurch gekennzeichnet, dass**
das Separieren des gelben Farbstoffes durch gerührtes Suspendieren in Dichlormethan bei Lichtausschluss und unter Argonatmosphäre erfolgt, wobei die gelbe Fraktion in Lösung geht und die beiden blau-violetten Farbstoffe als weiterverwendbares Feststoffgemisch auftreten.

4. Mikrobiologisches Verfahren nach Anspruch 3,
**dadurch gekennzeichnet, dass**
das Isolieren der beiden Farbstoffe aus dem Feststoffgemisch durch Suspendieren in einem Lösungsmittelgemisch Dichlormethan / Methanol (6+1), Absetzen in einem Zeitraum von ca. 12 h, Abdekantieren des Überstandes und Säulen-Chromatographieren des Überstandes über Kieselgel mit Dichlormethan / Methanol (6+1) erfolgt.

5. Mikrobiologisches Verfahren nach einem der Ansprüche 1 bis 4,
**dadurch gekennzeichnet, dass**
insbesondere der isolierte Farbstoff Violacein durch präparatives Hochdruck-Flüssigkeitschromatographieren oder durch isothermes Umkristallisieren aus Methanol / n-Hexan gereinigt wird.

6. Mikrobiologisches Verfahren nach einem der Ansprüche 1 bis 5,
**dadurch gekennzeichnet, dass**
das Kultivieren des marinen Sedimentbakteriums _{"}Black Beauty" durch Fermentieren in großtechnischem Rahmen erfolgt.

## Claims

1. A microbiological method for biosynthesis of the natural blue-violet dyes violacein und deoxyviolacein from a pigment-producing bacterium by culturing the bacterium, centrifuging the cell mass, extracting a crude dye extract from the cell mass and isolating the dyes from the crude dye extract,
**characterized in that**
the newly discovered marine sediment bacterium *Pseudoalteromonas species* strain "Black Beauty" (first deposited under the Budapest Treaty for the purposes of patent proceedings under the input number DSM 13623 with the Deutsche Sammlung von Mikroorganismen und Zellkulturen GmbH [German Collection of Microorganisms and Cell Cultures, Inc.], Braunschweig, Germany) is used as the pigment-producing bacterium, whereby a yellow dye occurs as a minor byproduct, which is separated by further extraction of the crude dye extract with dicholoromethane.

2. The microbiological method according to Claim 1,
**characterized in that**
the crude dye extract is extracted from the cell mass by slurrying with hot methanol, sedimentation of the cell mass over a period of approximately two hours and decanting off the supernatant with the methanolic extracts thereby formed in several repetitions, removing the solvent from the combined methanolic extracts on a rotary evaporator and drying in an oil pump vacuum.

3. The microbiological method according to Claim 1 or 2,
**characterized in that**
the yellow dye is separated by stirred suspension in dichloromethane in the absence of light an under an argon atmosphere, whereby the yellow fraction goes into solution and the two blue-violet dyes appear as a reusable mixture of solids.

4. Microbiological method according to Claim 3,
**characterized in that**
the two dyes are isolated from the mixture of solids by suspending in a solvent mixture of dichloromethane/methanol (6+1), sedimenting over a period of approximately twelve hours, decanting off the supernatant and performing column chromatography on the supernatant over silica gel using dichloromethane/methanol (6+1).

5. Microbiological method according to any one of Claims 1 through 4,
**characterized in that**
the isolated dye violacein in particular is purified by preparative high-pressure liquid chromatography or by isothermal recrystallization from methanol/n-hexane.

6. Microbiological method according to any one of Claims 1 through 5,
**characterized in that**
the "Black Beauty" marine sediment bacterium is cultured by fermenting in large-scale industrial frames.

## Revendications

1. Procédé microbiologique pour la biosynthèse des colorants bleu-violacé naturels violacéine et désoxyviolacéine à partir d'une bactérie productrice de pigments, consistant à mettre ladite bactérie en culture, à récupérer la masse cellulaire par centrifugation, à extraire un produit colorant brut de ladite masse cellulaire et à isoler les colorants contenus dans le produit colorant brut,
**caractérisé en ce que** l'on
utilise en tant que bactérie productrice de pigments la bactérie *Pseudoalteromonas spezies* souche "Black Beauty" qui a été découverte récemment (dépôt initial selon le traité de Budapest aux fins de la procédure en matière de brevets sous le numéro d'ordre DSM 13623 auprès de la Deutsche Sammlung von Mikroorganismen und Zellkulturen GmbH, Braunschweig, Allemagne), un colorant jaune étant obtenu comme sous-produit en quantités minimes, lequel est séparé par une extraction supplémentaire du produit colorant brut avec du dichlorométhane.

2. Procédé microbiologique selon la revendication 1,
**caractérisé en ce que**
l'extraction du produit colorant brut de la masse cellulaire est réalisée en mettant cette dernière en suspension dans du méthanol chaud, puis en laissent la masse cellulaire se sédimenter pendant environ 2 heures, puis en décantant, à plusieurs reprises, le surnageant qui contient les extraits méthanoliques, puis en réunissant les extraits méthanoliques et en enlevant le solvant au vaporisateur rotatif puis en procédant au séchage sous vide primaire.

3. Procédé microbiologique selon les revendications 1 ou 2,
**caractérisé en ce que**
la séparation du colorant jaune est réalisée par mise en suspension dans du dichlorométhane à l'abri de la lumière, sous atmosphère d'argon et sous agitation, la fraction jaune étant ainsi dissoute alors que les deux colorants bleu-violacé peuvent être récupérés sous forme d'un mélange de solides pour un traitement ultérieur.

4. Procédé microbiologique selon la revendication 3,
**caractérisé en ce que**
l'isolation des deux colorants contenus dans le mélange de solides est réalisée en le mettant en suspension dans un mélange de solvants constitué de dichlorométhane / méthanol (6+1) ; en laissent les solides se sédimenter durant environ 12 h ; en décantant le surnageant pour soumettre ce dernier à la chromatographie sur colonne de gel de silice avec du dichlorométhane / méthanol (6+1).

5. Procédé microbiologique selon l'une quelconque des revendication 1 à 4,
**caractérisé en ce que**
notamment le colorant violacéine est purifié, suite à son isolation, par chromatographie liquide à haute pression préparative ou par recristallisation isotherme dans du méthanol / n-hexane.

6. Procédé microbiologique selon l'une quelconque des revendication 1 à 5,
**caractérisé en ce que**
la culture de la bactérie "Black beauty" issue de sédiments marins est réalisée par fermentation à l'échelle industrielle.
